# EUROPÄISCHE PATENTANMELDUNG

(11) **EP 1 700 864 A1**
(43) Veröffentlichungstag der Anmeldung: **13.09.2006**
(21) Anmeldenummer: 06003718.1
(22) Anmeldetag: 23.02.2006
(51) Int. Cl.: C07K 14/245, C12N 15/63, C12P 19/40

(54) **Verfahren zur fermentativen Herstellung von S-Adenosylmethionin**

(30) Priorität: 03.03.2005 DE 102005009751
(71) Anmelder: Consortium für elektrochemische Industrie GmbH, 81737 München (DE)
(72) Erfinder: Schlösser, Thomas, Dr., 82515 Wolfratshausen (DE); Leonhartsberger, Susanne, Dr., 80634 München (DE); Flinspach, Renate, 83623 Dietramszell (DE)
(74) Vertreter: Potten, Holger

(57) **Zusammenfassung**

Die Erfindung betrifft einen Mikroorganismenstamm, der S-Adenosylmethionin sekretiert, dadurch gekennzeichnet, dass er eine erhöhte Aktivität des cmr (mdfA)-Genprodukts aufweist.

## Beschreibung

Die Erfindung betrifft ein Verfahren zur fermentativen Herstellung von S-Adenosylmethionin.

S-Adenosylmethionin (SAM) ist der wichtigste Methylgruppendonor im Stoffwechsel und findet im Pharmabereich Verwendung bei der Behandlung von Depressionen, Erkrankungen der Leber und Arthritis. Beschriebene Verfahren zur SAM-Herstellung umfassen die Anzucht von Hefen (Schlenk und DePalma, J. Biol. Chem. 229, 1037-1050 (1957), Schlenk et al., Enzymologia 29, 283-298 (1965), Shiozaki et al., J. Biotechnol. 4, 345-354 (1986), Shiozaki et al., Agric. Biol. Chem. 53, 3269-3274 (1989)) in Gegenwart der Vorstufe Methionin und die chromatographische Aufreinigung des gebildeten SAM nach Extraktion aus dem Zelllysat (US4562149). Charakteristisch für die Herstellung von SAM mit einer Hefe ist, dass SAM intrazellulär produziert und gespeichert wird. Um eine weitere Aufarbeitung von SAM durchzuführen, müssen die Zellen zuerst aufgebrochen werden, wie es z.B. in EP162323 (Beispiel 2) oder in DE3329218 (Beispiel 1) beschrieben wurde. Neben chemischen Aufschlussmethoden sind auch mechanische Methoden mittels French Press oder Hochdruckhomogenisation oder auch ein thermisches Verfahren (beschrieben in EP1091001, Beispiel 1) möglich.

Im Gegensatz zur SAM-Produktion mit Hefen ist im Stand der Technik ein bakterielles SAM-Produktionsverfahren mit Escherichia coli (E. coli) beschrieben, bei dem die Bakterien das produzierte SAM aus der Zelle in das Kulturmedium ausschleusen (EP 1 457 569 A1). Dieses fermentative SAM-Produktionsverfahren weist gegenüber dem bestehenden Hefe-Verfahren einen deutlichen Vorteil auf, da es eine selektive Sekretion des gebildeten SAM in den Kulturüberstand beschreibt und damit eine Vereinfachung des Aufreinigungsverfahrens ermöglicht. Im Kulturüberstand befinden sich nur wenige Substanzen, weshalb eine Sekretion von SAM schon einen ersten Aufreinigungsschritt darstellt und die weitere Aufarbeitung deutlich erleichtert. Bei diesem Verfahren zur extrazellulären SAM-Produktion wird eine SAM-Synthetase verwendet.

Aufgabe der vorliegenden Erfindung ist es, einen Mikroorganismenstamm zur Verfügung zu stellen, der eine im Vergleich zum Stand der Technik gesteigerte Produktion von SAM ermöglicht. Eine weitere Aufgabe ist es, ein Verfahren für die Herstellung von SAM mittels des erfindungsgemäßen Mikroorganismenstammes zur Verfügung zu stellen.

Die erstgenannte Aufgabe wird gelöst durch einen Mikroorganismenstamm der S-Adenosylmethionin sekretiert, dadurch gekennzeichnet, dass er eine erhöhte Aktivität des cmr (mdfA)-Genprodukts aufweist.

Der erfindungsgemäße Mikroorganismenstamm ist herstellbar aus einem Ausgangsstamm und weist eine gegenüber dem Ausgangsstamm erhöhte Aktivität des cmr (mdfA)-Genprodukts auf.

Ein Mikroorganismenstamm weist vorzugsweise dann eine gegenüber dem Ausgangsstamm erhöhte Aktivität des cmr (mdfA)-Genproduktes auf, wenn eine Zelle des Stammes im Vergleich zu einer Zelle eines Wildtyp-Mikroorganismenstammes mit cmr (mdfA)-Genprodukt eine um mindestens den Faktor 2, bevorzugt um mindestens den Faktor 5, erhöhte Aktivität des cmr (mdfA)-Genproduktes aufweist. Experimente zur Bestimmung der Aktivität des cmr (mdfA)-Genprodukts sind in der wissenschaftlichen Literatur beschrieben (Edgar und Bibi, J. Bacteriol. 179, 2274-2280).

Das cmr (mdfA)-Gen von E. coli wurde 1996 als Chloramphenicol-Export-Protein Cmr identifiziert (Nilsen et al., J. Bacteriol. 178, 3188-3193). 1997 wurde das cmr (mdfA)-Gen erneut als Multidrug-Efflux-Protein MdfA mit einem breiten Substratspektrum beschrieben (Edgar und Bibi, J. Bacteriol. 179, 2274-2280). Das Cmr (MdfA)-Protein gehört zur Familie der MF(S)[Major Facilliator (Superfamily)]-Transporter und schleust sowohl lipophile, ungeladene Substrate wie z. B. Chloramphenicol oder Erythromycin als auch lipophile, positiv geladene Substrate, wie z.B. Ethidiumbromid, Doxorubicin oder Benzalkonium im Austausch mit H⁺-Ionen aus der Zelle (siehe Review von Bibi et al., J. Mol. Microbiol. Biotechnol. 3, 171-177 (2001)). Obwohl das Cmr (MdfA)-Protein ein breites Substratspektrum besitzt, ist es für den Fachmann überraschend, dass Cmr (MdfA) als SAM-Export-Protein fungieren kann, da SAM keine strukturellen Gemeinsamkeiten mit den bisher beschriebenen Substraten besitzt. Weiterhin zeigen sowohl die Nukleinsäuresequenz von cmr (mdfA) als auch die Aminosäuresequenz des Cmr (MdfA)-Proteins keine Homologien zu den bisher bekannten SAM-Transportgenen und SAM-Transportproteinen aus der Hefe und des Menschen. Eine Vorhersage, ob eine Substanz als Substrat für das Cmr (MdfA)-Protein fungieren kann, ist auch aufgrund des weitgehend unbekannten Transportmechanismus nicht möglich. Weiterhin ist für den Fachmann völlig überraschend, dass im erfindungsgemäßen Mikroorganismenstamm mit SAM ein stark hydrophiles, positiv geladenes und gleichzeitig zelleigenes Molekül aus der Zelle heraustransportiert wird.

Die Erfindung betrifft daher auch die Verwendung des cmr (mdfA)-Genprodukts als Export-Protein bei der Herstellung von SAM.

Das cmr (mdfA)-Gen und das cmr (mdfA)-Genprodukt (Cmr (MdfA)-Protein) sind durch die Sequenzen SEQ ID No. 1 beziehungsweise SEQ ID No. 2 charakterisiert. Im Rahmen der vorliegenden Erfindung sind als cmr (mdfA)-Gene auch solche Gene aufzufassen, die für ein Protein mit Chloramphenicol-Export- oder Multidrug-Efflux-Aktivität kodieren und mit dem Algorithmus BESTFIT (GCG Wisconsin Package, Genetics Computer Group (GCG) Madison, Wisconsin) eine Sequenzidentität von größer 30 % zu SEQ ID No. 1 aufweisen. Bevorzugt ist eine Sequenzidentität von größer 50 % zu SEQ ID No. 1. Besonders bevorzugt ist eine Sequenzidentität von größer 70 % zu SEQ ID No. 1.

Ebenso sind Proteine mit Chloramphenicol-Export- oder Multidrug-Efflux-Aktivität und mit einer Sequenzidentität von größer 15 % zu SEQ ID No. 2 Algorithmus BESTFIT (GCG Wisconsin Package, Genetics Computer Group (GCG) Madison, Wisconsin) als Cmr (MdfA)-Proteine aufzufassen. Bevorzugt ist eine Sequenzidentität größer 30 % zu SEQ ID No. 2. Besonders bevorzugt ist eine Sequenzidentität größer 60 % zu SEQ ID No. 2.

Somit sind als cmr (mdfA)-Gene auch Allelvarianten des cmr (mdfA)-Gens zu verstehen, insbesondere funktionelle Varianten, die sich durch Deletion, Insertion oder Substitution von Nukleotiden aus der in SEQ ID No. 1 dargestellten Sequenz ableiten, wobei die enzymatische Aktivität des jeweiligen Genprodukts jedoch zumindest erhalten bleiben muss.

Erfindungsgemäße Mikroorganismen können mit Standardtechniken der Molekularbiologie erzeugt werden.

Als Ausgangsstämme sind prinzipiell alle Mikroorganismen geeignet, die den Biosyntheseweg für SAM aufweisen, rekombinanten Verfahren zugänglich sind und durch Fermentation kultivierbar sind. Solche Mikroorganismen können Pilze, Hefen oder Bakterien sein. Bevorzugt handelt es sich um Bakterien der phylogenetischen Gruppe der Eubacteria. Besonders bevorzugt sind Mikroorganismen der Familie Enterobacteriaceae und insbesondere der Art Escherichia coli.

Die Erhöhung der Aktivität des cmr (mdfA)-Genprodukts im erfindungsgemäßen Mikroorganismus wird beispielsweise durch eine verstärkte Expression des cmr (mdfA)-Gens erreicht. Dabei kann die Kopienzahl des cmr (mdfA)-Gens in einem Mikroorganismus erhöht sein und/oder es kann durch geeignete Promotoren die Expression des cmr (mdfA)-Gens gesteigert sein. Unter verstärkter Expression ist dabei vorzugsweise zu verstehen, dass das cmr (mdfA)-Gen mindestens doppelt so stark exprimiert wird wie im Ausgangsstamm. Unter einer erhöhten Kopienzahl das cmr (mdfA)-Gens ist vorzugsweise zu verstehen, dass im Vergleich zum Ausgangsstamm zusätzlich mindestens eine chromosomal und/oder plasmidcodierte Kopie des cmr (mdfA)-Gens verwendet wird.

Die Erhöhung der Kopienzahl des cmr (mdfA)-Gens in einem Mikroorganismus kann mit dem Fachmann bekannten Methoden vorgenommen werden. So kann zum Beispiel das cmr (mdfA)-Gen in Plasmid-Vektoren mit mehrfacher Kopienzahl pro Zelle (z. B. pUC19, pBR322, pACYC184 für E. coli) kloniert und in den Mikroorganismus eingebracht werden. Alternativ kann das cmr (mdfA)-Gen mehrfach ins Chromosom eines Mikroorganismus integriert werden. Als Integrationsverfahren können die bekannten Systeme mit temperenten Bakteriophagen, integrative Plasmide oder die Integration über homologe Rekombination genutzt werden.

Bevorzugt ist die Erhöhung der Kopienzahl durch Klonierung eines cmr (mdfA)-Gens in Plasmid-Vektoren unter der Kontrolle eines Promotors. Besonders bevorzugt ist die Erhöhung der Kopienzahl in E. coli durch Klonierung eines cmr (mdfA)-Gens in einem pACYC-Derivat.

Als Kontrollregion für die Expression eines plasmid-kodierten cmr (mdfA)-Gens kann die natürliche Promotor- und Operatorregion des Gens dienen.

Die verstärkte Expression eines cmr (mdfA)-Gens kann jedoch auch mittels anderer Promotoren erfolgen. Entsprechende Promotorsysteme wie beispielsweise in E. coli der konstitutive GAPDH-Promotor des gapA-Gens oder die induzierbaren lac-, tac-, trc-, lambda-, ara- oder tet-Promotoren sind dem Fachmann bekannt. Solche Konstrukte können in an sich bekannter Weise auf Plasmiden oder chromosomal verwendet werden.

Des Weiteren kann eine verstärkte Expression dadurch erreicht werden, dass Translationsstartsignale, wie z. B. die Ribosomenbindestelle oder das Startcodon des Gens, in optimierter Sequenz auf dem jeweiligen Konstrukt vorhanden sind oder dass gemäß der Codon-Nutzung seltene Codons gegen häufiger vorkommende Codons ausgetauscht werden.

Mikroorganismenstämme mit den genannten Modifikationen sind bevorzugte Ausführungen der Erfindung.

Die Klonierung eines cmr (mdfA)-Gens in Plasmid-Vektoren erfolgt beispielsweise durch spezifische Amplifikation mittels der Polymerase-Ketten-Reaktion unter Einsatz von spezifischen Primern, die das komplette cmr (mdfA)-Gen erfassen, und anschließende Ligation mit Vektor-DNA-Fragmenten.

Als bevorzugte Vektoren für die Klonierung eines cmr (mdfA)-Gens werden Plasmide verwendet, die bereits Promotoren zur verstärkten Expression enthalten, beispielsweise den konstitutiven GAPDH-Promotor des gapA-Gens von E. coli.

Des weiteren sind Vektoren besonders bevorzugt, die bereits ein Gen/Allel enthalten, dessen Einsatz zu einer verstärkten Biosynthese von SAM führt, wie beispielsweise ein Rattenleber-SAM-Synthetase (RLSS)-Allel, das metK-Gen (beschrieben in EP 0 647 712 A1 und EP 1 457 569 A1) oder Kombinationen aus mehreren SAM-Synthetasen. Selbstverständlich können auch Plasmide verwendet werden, die mehrere Kopien eines SAM-Synthetase-Gens enthalten. Solche Vektoren ermöglichen die direkte Herstellung von erfindungsgemäßen Mikroorganismenstämmen mit hoher SAM-Überproduktion aus einem beliebigen Mikroorganismenstamm.

Die Erfindung betrifft somit auch ein Plasmid, das dadurch gekennzeichnet ist, dass es ein SAM-Synthetase-Gen sowie ein cmr (mdfA)-Gen unter der Kontrolle eines Promotors enthält. Ein solches Plasmid kann auch Kombinationen aus SAM-Synthetase-Genen verschiedener Organismen oder mehrere Kopien eines SAM-Synthetase-Gens enthalten.

Durch eine gängige Transformationsmethode (z.B. Elektroporation) werden die cmr (mdfA)-haltigen Plasmide in Mikroorganismen eingebracht und beispielsweise mittels Antibiotika-Resistenz auf plasmid-tragende Klone selektiert.

Die Erfindung betrifft somit auch Verfahren zur Herstellung eines erfindungsgemäßen Mikroorganismenstammes, dadurch gekennzeichnet, dass in einen Ausgangsstamm ein erfindungsgemäßes Plasmid eingebracht wird.

Besonders bevorzugt als Stämme für die Transformation mit einem erfindungsgemäßen Plasmid sind solche, die bereits Allele aufweisen, die ebenfalls eine SAM-Produktion begünstigen können, wie beispielsweise
- Allele, die eine verbesserte SAM-Produktion bewirken, wie das RLSS-Gen oder das metK-Gen (wie beispielsweise in EP 1 457 569 A1 beschrieben)
- oder Gene, die eine verbesserte Methionin-Aufnahme bewirken, wie beispielsweise das metNIQ-Operon aus E. coli (Merlin et al., J. Bacteriol. 184, 5513-5517 (2002); Gäl et al., J. Bacteriol. 184, 4930-4932 (2002); Zhang et al., Arch. Microbiol. 180, 88-100 (2003))
- oder Gene, die eine verbesserte zelleigene Methionin-Synthese begünstigen, wie beispielsweise ein verbessertes Homoserin-Transsuccinylase-Gen (JP2000139471A, DE-A-10247437, DE-A-10249642)

Die Produktion von SAM mit Hilfe eines erfindungsgemäßen Mikroorganismenstammes erfolgt in einem Fermenter nach an sich bekannten Verfahren.

Die Erfindung betrifft somit auch ein Verfahren zur Herstellung von SAM, welches dadurch gekennzeichnet ist, dass ein erfindungsgemäßer Mikroorganismenstamm in einem Fermentationsmedium fermentiert wird und dabei SAM in das Fermentationsmedium sekretiert wird. Vorzugsweise wird das produzierte SAM aus dem Fermentationsansatz abgetrennt.

Die Anzucht des Mikroorganismenstammes im Fermenter erfolgt als kontinuierliche Kultur, als batch-Kultur oder vorzugsweise als fed-batch-Kultur. Besonders bevorzugt wird eine Kohlenstoff-Quelle während der Fermentation kontinuierlich zudosiert.

Als Kohlenstoff-Quelle dienen vorzugsweise Zucker, Zuckeralkohole oder organische Säuren. Besonders bevorzugt werden im erfindungsgemäßen Verfahren als Kohlenstoff-Quellen Glukose, Laktose oder Glycerin eingesetzt.

Bevorzugt ist die Dosierung der Kohlenstoff-Quelle in einer Form, die gewährleistet, dass der Gehalt an Kohlenstoff-Quelle im Fermenter während der Fermentation in einem Bereich von 0,1 - 50 g/l gehalten wird. Besonders bevorzugt ist ein Bereich von 0,5 - 10 g/l.

Als Stickstoff-Quelle werden im erfindungsgemäßen Verfahren vorzugsweise Ammoniak, Ammoniumsalze oder Proteinhydrolysate verwendet. Bei Verwendung von Ammoniak als Korrekturmittel zur pH-Statisierung wird während der Fermentation regelmäßig diese Stickstoff-Quelle nachdosiert.

Als weitere Medienzusätze können Salze der Elemente Phosphor, Chlor, Natrium, Magnesium, Stickstoff, Kalium, Calcium, Eisen und in Spuren (d. h. in µM Konzentrationen) Salze der Elemente Molybdän, Bor, Kobalt, Mangan, Zink und Nickel zugesetzt werden.

Des Weiteren können organische Säuren (z. B. Acetat, Citrat), Aminosäuren (z. B. Isoleucin) und Vitamine (z. B. B₁, B₁₂) dem Medium zugesetzt werden.

Als komplexe Nährstoffquellen können z. B. Hefeextrakt, Maisquellwasser, Sojamehl oder Malzextrakt zum Einsatz kommen.

Außerdem kann dem Medium L-Methionin oder D/L-Methionin als spezifische Vorstufe für die SAM-Synthese in einer Konzentration zwischen 0,05 und 25 g/l zugesetzt werden. Bevorzugt ist der Zusatz von L-Methionin oder D/L-Methionin in der Konzentration zwischen 1 und 7 g/l.

Zusätzlich wird in einem besonders bevorzugten erfindungsgemäßen Verfahren dem Medium L-Methionin oder D,L-Methionin kontinuierlich während der Kultivierung zudosiert. Bevorzugt ist eine kontinuierliche Zudosierung von L-Methionin oder D/L-Methionin zwischen 0,05 g und 10 g pro Stunde. Besonders bevorzugt ist eine kontinuierliche Zudosierung von L-Methionin oder D/L-Methionin zwischen 0,1 g und 2 g pro Stunde.

Die Inkubationstemperatur für mesophile Mikroorganismen beträgt vorzugsweise 15 - 45 °C, besonders bevorzugt 30 - 37 °C.

Die Fermentation wird vorzugsweise unter aeroben Wachstumsbedingungen durchgeführt. Der Sauerstoffeintrag in den Fermenter erfolgt mit Druckluft oder mit reinem Sauerstoff.

Der pH-Wert des Fermentationsmediums liegt während der Fermentation bevorzugt im pH-Bereich von 5,0 bis 8,5, besonders bevorzugt ist ein pH-Wert von 7,0.

Die Inkubation des Stammes erfolgt vorzugsweise unter aeroben Kultivierungsbedingungen über einen Zeitraum von 16 - 150 h und im Bereich der für den jeweiligen Stamm optimalen Wachstumstemperatur. Besonders bevorzugt sind Kultivierungszeiten zwischen 20 und 48 h.

Die Abtrennung von SAM aus dem Kulturmedium kann nach dem Fachmann bekannten Verfahren, wie Zentrifugation des Mediums zur Abtrennung der Zellen, Querstromfiltration zur Abtrennung von Proteinen und anschließende chromatographische Reinigung, Konzentrierung, Formulierung oder Komplexierung des Produktes, erfolgen.

Der Nachweis und die Quantifizierung des im erfindungsgemäßen Verfahren produzierten SAM erfolgt beispielsweise mittels Chromatographie (z.B. HPLC).

Die folgenden Beispiele dienen der weiteren Erläuterung der Erfindung.

### Beispiel 1: Konstruktion des Plasmids pFL242

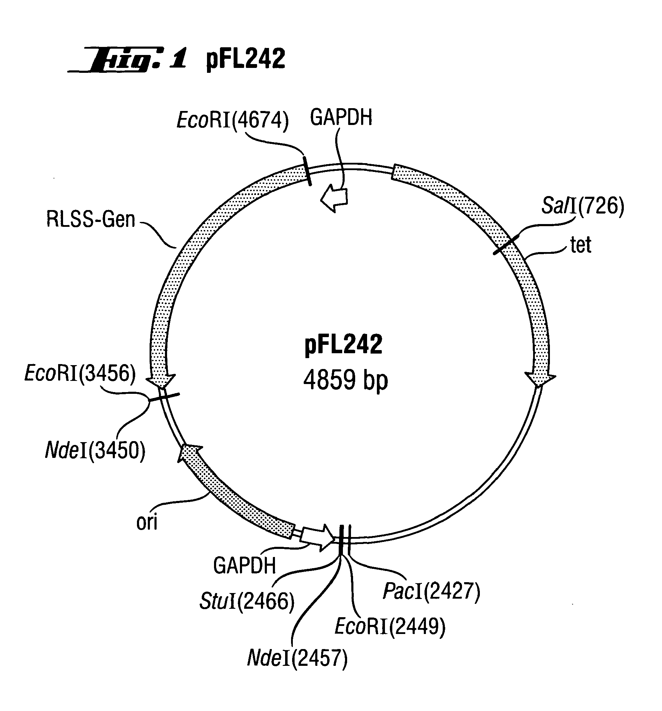

Zur Klonierung des GAPDH-Promotor-RLSS-Fragmentes wurde das Plasmid pKP504 mit Hilfe der Restriktionsendonuklease *SspI* linearisiert. Die Herstellung des Plasmids pKP504 ist im Patent EP-A-1 457 569, Beispiel 4, beschrieben. Anschließend folgte eine Dephosphorylierung des linearisierten Plasmids mit Hilfe einer alkalischen Phosphatase (Roche, Mannheim). Abschließend wurde vor der Ligation eine Reinigung des linearisierten Vektors unter Verwendung eines QIAquick Nucleotide Removal Kit (Qiagen, Hilden) nach Herstellerangaben durchgeführt.
Das GAPDH-Promotor-RLSS-Fragment wurde aus dem SAM-Produktionsplasmid pMSRLSSk (EP 1 457 569 A1) mit Hilfe der Restriktionsendonukleasen Ecl136II und StuI isoliert. Nach Reinigung des Fragmentes über eine Agarose-Gelelektrophorese mit anschließender Gel-Extraktion (QIAquick Gel Extraktion Kit, Qiagen, Hilden) erfolgte eine Ligation des GAPDH-Promotor-RLSS-Fragmentes und des *Ssp*I-linearisierten Vektors pKP504 mittels einer T4-DNA-Ligase (Roche, Mannheim) nach Herstellerangaben.
Die Transformation von E. coli-Zellen des Stammes DH5α (Invitrogen, Karlsruhe) mit dem Ligationsansatz wurde mittels Elektroporation in einer dem Fachmann bekannten Art und Weise durchgeführt. Der Transformationsansatz wurde auf LB-Tetracyclin-Agarplatten (10 g/l Trypton, 5 g/l Hefeextrakt, 5 g/l NaCl, 15 g/l Agar, 20 mg/l Tetracyclin) aufgebracht und über Nacht bei 37°C inkubiert.
Die gewünschten Transformanten wurden nach einer Plasmidisolierung mittels eines QIAprep Spin Miniprep Kit (Qiagen, Hilden) durch eine Restriktionsanalyse identifiziert. In dem auf diese Weise erhaltenen Plasmid pFL242 (Fig. 1) ist nunmehr die Klonierung eines weiteren Gens unter Kontrolle des konstitutiven GAPDH-Promotors des gapA-Gens aus E. coli möglich. Das Plasmid pFL242, das für die Ausführung der Beispiele verwendet wurde, wurde am 17.02.2005 bei der DSMZ (Deutsche Sammlung für Mikroorganismen und Zellkulturen GmbH, D-38142 Braunschweig) unter der Nummer DSM 17142 gemäß Budapester Vertrag hinterlegt.

### Beispiel 2: Konstruktion des Plasmids pFL274

### A. Amplifizierung des cmr (mdfA)-Gens

Das cmr (mdfA)-Gen aus E. coli wurde mittels der Polymerase-Ketten-Reaktion (PCR) unter Verwendung der Taq-DNA-Polymerase nach gängiger, dem Fachmann bekannter Praxis amplifiziert. Als Matrize diente die chromosomale DNA des E. coli-Wildtypstammes W3110 (ATCC 27325). Als Primer wurden die Oligonukleotide cmr for (SEQ ID No: 3) mit der Sequenz 
5´-AAA AGG CCT TGC ATG CAA AAT AAA TTA GCT TC-3´
StuI
 und cmr rev (SEQ ID No: 4) mit der Sequenz 
5'-CCC TTA ATT AAA CCA GAT TGA CGA CCA TCA C-3'
PacI
 verwendet.
Das bei der PCR erhaltene DNA-Fragment mit einer Länge von ca. 1,3 kb wurde anschließend mittels eines DNA-Adsorptionssäulchens des QIAprep Spin Miniprep Kits (Qiagen, Hilden) nach Herstellerangaben gereinigt.

### B. Klonierung des cmr (mdfA)-Gens in den Vektor pFL242

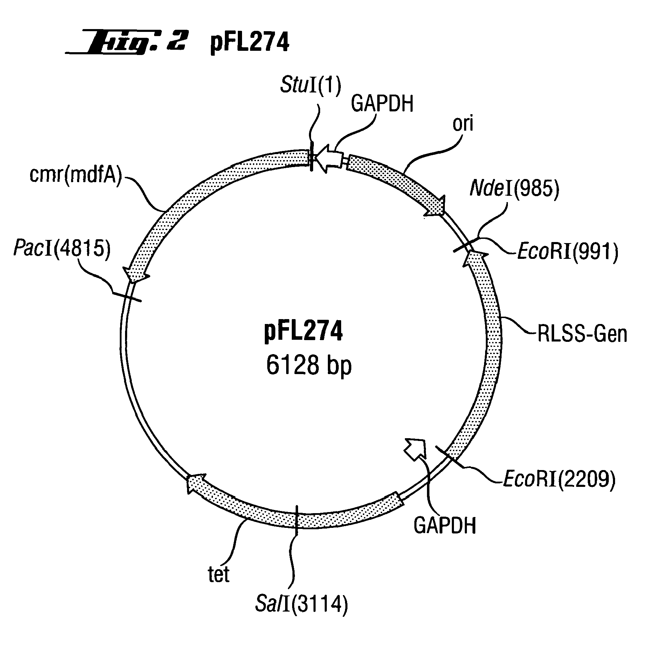

In das PCR-Fragment wurden über die Primer cmr for und cmr rev zwei Schnittstellen für die Restriktionsendonukleasen StuI und PacI eingeführt. Das gereinigte PCR-Fragment wurde mit den Restriktionsendonucleasen *Stu*I (Roche, Mannheim) und PacI (New England Biolabs, Frankfurt am Main) unter den vom Hersteller angegebenen Bedingungen geschnitten, über ein Agarosegel aufgetrennt und dann mittels des QIAquick Gel Extraction Kits (Qiagen, Hilden) nach Herstellerangaben aus dem Agarosegel isoliert.
Zur Klonierung des cmr (mdfA)-Gens wurde der Vektor pFL242 mit den Restriktionsenzymen StuI und PacI unter den vom Hersteller angegebenen Bedingungen geschnitten. Das Plasmid wurde anschließend durch Behandlung mit Alkalischer Phosphatase (Roche, Mannheim) an den 5'-Enden dephosphoryliert und dann wie das PCR-Fragment mittels QIAquick Gel Extraction Kit (Qiagen, Hilden) gereinigt. Die Ligation des PCR-Fragments mit dem geschnittenen und dephosphorylierten Vektor erfolgte nach Herstellerangaben unter Verwendung der T4-DNA-Ligase (Roche, Mannheim). Die Transformation von E. coli-Zellen des Stammes W3110 (ATCC 27325) mit dem Ligationsansatz wurde mittels Elektroporation in einer dem Fachmann bekannten Art und Weise durchgeführt. Der Transformationsansatz wurde auf LB-Tetracyclin-Agarplatten (10 g/l Trypton, 5 g/l Hefeextrakt, 5 g/l NaCl, 15 g/l Agar, 20 mg/l Tetracyclin) ausgebracht und über Nacht bei 37°C inkubiert.
Die gewünschten Transformanten wurden nach einer Plasmidisolierung mittels eines QIAprep Spin Miniprep Kit (Qiagen, Hilden) durch eine Restriktionsanalyse identifiziert und die Fehlerfreiheit durch Sequenzanalyse bestätigt.
Im dem auf diese Weise erhaltenen Plasmid pFL274 (Fig. 2) ist das cmr (mdfA)-Gen unter Kontrolle des GAPDH-Promotors.

### Beispiel 3: Herstellung eines Produzenten von S-Adenosylmethionin

Das in Beispiel 2 beschriebene Plasmid pFL274 wurde mittels CaCl₂-Methode zur Transformation des E. coli-Stammes W3110 (ATCC 27325) eingesetzt und nach Selektion auf LB-Agarplatten mit 20 mg/l Tetracyclin wurde das Plasmid aus einem der Transformanten reisoliert, mit Restriktionsendonucleasen gespalten und überprüft. Dieser Stamm wird als W3110/pFL274 bezeichnet und ist für die Produktion von SAM geeignet.

### Beispiel 4: Fermentative Produktion von S-Adenosylmethionin

### A. Produktion von SAM

Für die fermentative Produktion von SAM wurde der Stamm W3110/pFL274 verwendet. Als Vergleich dienten zum einen der Wildtyp-Stamm W3110 (ATCC 27325) ohne Plasmid und zum anderen der Stamm W3110/pMSRLSSk, die unter denselben Bedingungen kultiviert wurden. W3110/pMSRLSSk wurde analog Beispiel 3 aus W3110 und dem Plasmid pMSRLSSk hergestellt.
Für die Anzucht wurde folgendes Medium verwendet: für 1 l Medium: CaCl₂ x 2 H₂O 0,0147 g, MgSO₄ x 7 H₂O 0,3 g, Na₂MoO₄ x 2 H₂O 0, 15 mg, H₃BO₃ 2, 5 mg, CoCl₂ x 6 H₂O 0, 7 mg, CuSO₄ x 5 H₂O 0,25 mg, MnCl₂ x 4 H₂O 1,6 mg, ZnSO₄ x 7 H₂O 0,3 mg, KH₂PO₄ 3,0 g, K₂HPO₄ 12,0 g, (NH₄)₂SO₄ 5 g, NaCl 0,6 g, FeSO₄ x 7 H₂O 0,002 g, Na₃-Citrat x 2 H₂O 1 g, Glucose 15 g, Trypton 1 g, Hefeextrakt 0,5 g.
Bei der Anzucht von W3110/pMSRLSSk und W3110/pFL274 wurde dem Medium 20 µg/ml Tetracyclin zugesetzt. Außerdem enthielt das Medium einen Zusatz von 0,5 g/l L-Methionin.
Als Vorkultur für die Produktionsanzucht wurden zunächst 3 ml Medium in einem Reagenzglasröhrchen mit dem jeweiligen Stamm beimpft und für 16 h bei 37°C und 150 rpm auf einem Schüttler inkubiert. Mit den auf diese Weise vorbereiteten Zellen wurden schließlich 20 ml desselben Mediums in einem 100 ml Erlenmeyerkolben auf eine OD₆₀₀ (Absorption bei 600 nm) von 0,1 beimpft. Die Inkubation der Produktionskulturen erfolgte bei 37 °C und 150 rpm auf einem Schüttler bis zu 48 h. Nach 24 h und 48 h wurden Proben entnommen und die Zellen durch Zentrifugation vom Kulturmedium abgetrennt.

### B. Quantifizierung des produzierten SAM

Das im Kulturüberstand enthaltene SAM wurde mittels HPLC quantifiziert. Dabei wurde eine Develosil RP-Aqueous C 30- Säule, 5 µm, 250 * 4,6 mm (Phenomenex, Aschaffenburg) verwendet und 10 µL aufgebrachter Kulturüberstand mittels isokratischer Elution mit einem Fließmittel aus 3 ml 85 %iger H₃PO₄, auf 1 1 H₂O bei einer Flussrate von 0,5 ml/min bei Raumtemperatur aufgetrennt und mittels Diodenarray-Detektor bei einer Wellenlänge von 260 nm quantifiziert. Tabelle 1 zeigt die erzielten Gehalte von SAM im jeweiligen Kulturüberstand.

**Tabelle 1:**

| Stamm | S-Adenosylmethionin [mg/l] | |
|---|---|---|
| | 24 h | 48 h |
| W3110 | 0 | 0 |
| W3110/pMSRLSSk | 79 | 63 |
| W3110/pFL274 | 287 | 222 |

## Patentansprüche

1. Mikroorganismenstamm, der S-Adenosylmethionin sekretiert, **dadurch gekennzeichnet, dass** er eine erhöhte Aktivität des cmr (mdfA)-Genprodukts aufweist.

2. Mikroorganismenstamm gemäß Anspruch 1, **dadurch gekennzeichnet, dass** eine Zelle des Stammes im Vergleich zu einer Zelle eines Wildtyp-Mikroorganismenstammes mit cmr (mdfA)-Genprodukt eine um mindestens den Faktor 2, bevorzugt um mindestens den Faktor 5, erhöhte Aktivität des cmr (mdfA)-Genproduktes aufweist.

3. Mikroorganismenstamm gemäß Anspruch 1 oder 2, **dadurch gekennzeichnet, dass** das cmr (mdfA)-Genprodukt die Aminosäuresequenz SEQ ID No. 2 oder eine Sequenzidentität von größer 15 % zu SEQ ID No. 2 besitzt.

4. Mikroorganismenstamm gemäß Anspruch 1, 2 oder 3, **dadurch gekennzeichnet, dass** es sich um einen Stamm der Familie Enterobacteriaceae, vorzugsweise der Art Escherichia coli, handelt.

5. Plasmid, **dadurch gekennzeichnet, dass** es ein SAM-Synthetase-Gen sowie ein cmr (mdfA)-Gen mit einem Promotor enthält.

6. Verfahren zur Herstellung eines Mikroorganismenstammes gemäß einem der Ansprüche 1 bis 4, **dadurch gekennzeichnet, dass** in einen Ausgangsstamm ein Plasmid gemäß Anspruch 5 eingebracht wird.

7. Verfahren zur Herstellung von SAM, **dadurch gekennzeichnet, dass** ein Mikroorganismenstamm gemäß einem der Ansprüche 1 bis 4 in einem Fermentationsmedium fermentiert wird und dabei SAM in das Fermentationsmedium sekretiert wird.

8. Verfahren nach Anspruch 7, **dadurch gekennzeichnet, dass** SAM aus dem Fermentationsmedium abgetrennt wird.

9. Verwendung eines cmr (mdfA)- Genprodukts als Export-Protein bei der Herstellung von SAM.


SEQUENCE LISTING

<110> Consortium fuer elektrochemische Industrie GmbH

<120> Verfahren zur fermentativen Herstellung von
      S-Adenosylmethionin

<130> Co10431

<140>
<141>

<160> 4

<170> PatentIn Ver. 2.0

<210> 1
<211> 1233
<212> DNA
<213> Escherichia coli

<220>
<221> gene
<222> (1)..(1230)

<300>
<301> Edgar, Rotem
<302> MdfA, an Escherichia coli multidrug resistance protein
      with an extraordinarily broad spectrum of drug
      recognition

<303> J. Bacteriol.

<304> 179

<305> 7

<306> 2274-2280

<400> 1

atgcaaaata aattagcttc cggtgccagg cttggacgtc aggcgttact tttccctctc 60

tgtctggtgc tttacgaatt ttcaacctat atcggcaacg atatgattca acccggtatg 120

ttggccgtgg tggaacaata tcaggcgggc attgattggg ttcctacttc gatgaccgcg 180

tatctggcgg gcgggatgtt tttacaatgg ctgctggggc cgctgtcgga tcgtattggt 240

cgccgtccgg tgatgctggc gggagtggtg tggtttatcg tcacctgtct ggcaatattg 300

ctggcgcaaa acattgaaca attcaccctg ttgcgcttct tgcagggcat aagcctctgt 360

ttcattggcg ctgtgggata cgccgcaatt caggaatcct tcgaagaggc ggtttgtatc 420

aagatcaccg cgctgatggc gaacgtggcg ctgattgctc cgctacttgg tccgctggtg 480

ggcgcggcgt ggatccatgt gctgccctgg gaggggatgt ttgttttgtt tgccgcattg 540

gcagcgatct cctttttcgg tctgcaacga gccatgcctg aaaccgccac gcgtataggc 600

gagaaactgt cactgaaaga actcggtcgt gactataagc tggtgctgaa gaacggccgc 660

tttgtggcgg gggcgctggc gctgggattc gttagtctgc cgttgctggc gtggatcgcc 720

cagtcgccga ttatcatcat taccggcgag cagttgagca gctatgaata tggcttgctg 780

caagtgccta ttttcggggc gttaattgcg ggtaacttgc tgttagcgcg tctgacctcg 840

cgccgcaccg tacgttcgct gattattatg ggcggctggc cgattatgat tggtctattg 900

gtcgctgctg cggcaacggt tatctcatcg cacgcgtatt tatggatgac tgccgggtta 960

agtatttatg ctttcggtat tggtctggcg aatgcgggac tggtgcgatt aaccctgttt 1020

gccagcgata tgagtaaagg tacggtttct gccgcgatgg gaatgctgca aatgctgatc 1080

tttaccgttg gtattgaaat cagcaaacat gcctggctga acgggggcaa cggactgttt 1140

aatctcttca accttgtcaa cggaattttg tggctgtcgc tgatggttat ctttttaaaa 1200

gataaacaga tgggaaattc tcacgaaggg taa 1233

<210> 2

<211> 410

<212> PRT

<213> Escherichia coli

<300>

<301> Edgar, Rotem

<302> MdfA, an Escherichia coli multidrug resistance protein

with an extraordinarily broad spectrum of drug

recognition

<303> J. Bacteriol.

<304> 179

<305> 7

<306> 2274-2280

<313> 1 TO 410

<400> 2

Met Gln Asn Lys Leu Ala Ser Gly Ala Arg Leu Gly Arg Gln Ala Leu

1               5                   10                  15

Leu Phe Pro Leu Cys Leu Val Leu Tyr Glu Phe Ser Thr Tyr Ile Gly

            20                  25                  30

Asn Asp Met Ile Gln Pro Gly Met Leu Ala Val Val Glu Gln Tyr Gln

        35                  40                  45

Ala Gly Ile Asp Trp Val Pro Thr Ser Met Thr Ala Tyr Leu Ala Gly

    50                  55                  60

Gly Met Phe Leu Gln Trp Leu Leu Gly Pro Leu Ser Asp Arg Ile Gly

65                  70                  75                  80

Arg Arg Pro Val Met Leu Ala Gly Val Val Trp Phe Ile Val Thr Cys
                85                  90                  95

Leu Ala Ile Leu Leu Ala Gln Asn Ile Glu Gln Phe Thr Leu Leu Arg
            100                 105                 110

Phe Leu Gln Gly Ile Ser Leu Cys Phe Ile Gly Ala Val Gly Tyr Ala
        115                 120                 125

Ala Ile Gln Glu Ser Phe Glu Glu Ala Val Cys Ile Lys Ile Thr Ala
    130                 135                 140

Leu Met Ala Asn Val Ala Leu Ile Ala Pro Leu Leu Gly Pro Leu Val
145                 150                 155                 160

Gly Ala Ala Trp Ile His Val Leu Pro Trp Glu Gly Met Phe Val Leu
                165                 170                 175

Phe Ala Ala Leu Ala Ala Ile Ser Phe Phe Gly Leu Gln Arg Ala Met
            180                 185                 190

Pro Glu Thr Ala Thr Arg Ile Gly Glu Lys Leu Ser Leu Lys Glu Leu
        195                 200                 205

Gly Arg Asp Tyr Lys Leu Val Leu Lys Asn Gly Arg Phe Val Ala Gly
    210                 215                 220

Ala Leu Ala Leu Gly Phe Val Ser Leu Pro Leu Leu Ala Trp Ile Ala
225                 230                 235                 240

Gln Ser Pro Ile Ile Ile Ile Thr Gly Glu Gln Leu Ser Ser Tyr Glu
                245                 250                 255

Tyr Gly Leu Leu Gln Val Pro Ile Phe Gly Ala Leu Ile Ala Gly Asn
            260                 265                 270

Leu Leu Leu Ala Arg Leu Thr Ser Arg Arg Thr Val Arg Ser Leu Ile
        275                 280                 285

Ile Met Gly Gly Trp Pro Ile Met Ile Gly Leu Leu Val Ala Ala Ala
    290                 295                 300

Ala Thr Val Ile Ser Ser His Ala Tyr Leu Trp Met Thr Ala Gly Leu
305                 310                 315                 320

Ser Ile Tyr Ala Phe Gly Ile Gly Leu Ala Asn Ala Gly Leu Val Arg
                325                 330                 335

Leu Thr Leu Phe Ala Ser Asp Met Ser Lys Gly Thr Val Ser Ala Ala
            340                 345                 350

Met Gly Met Leu Gln Met Leu Ile Phe Thr Val Gly Ile Glu Ile Ser
        355                 360                 365

Lys His Ala Trp Leu Asn Gly Gly Asn Gly Leu Phe Asn Leu Phe Asn
    370                 375                 380

Leu Val Asn Gly Ile Leu Trp Leu Ser Leu Met Val Ile Phe Leu Lys
385                 390                 395                 400

Asp Lys Gln Met Gly Asn Ser His Glu Gly
                405                 410

<210> 3
<211> 32
<212> DNA
<213> Artificial Sequence

<220>
<223> Description of Artificial Sequence:
Oligonucleotide cmr for

<400> 3
aaaaggcctt gcatgcaaaa taaattagct tc 32

<210> 4
<211> 31
<212> DNA
<213> Artificial Sequence

<220>
<223> Description of Artificial Sequence:
Oligonucleotide cmr rev

<400> 4
cccttaatta aaccagattg acgaccatca c 31